(19) Europäisches Patentamt
European Patent Office
Office européen des brevets

(11) Veröffentlichungsnummer: **0 069 953**
**A1**

(12) # EUROPÄISCHE PATENTANMELDUNG

(21) Anmeldenummer: 82105965.6

(22) Anmeldetag: 03.07.82

(51) Int. Cl.³: **C 07 D 471/04**
C 07 D 491/147, A 61 K 31/53
//(C07D471/04, 221/00, 251/00)

(30) Priorität: 08.07.81 DE 3126837

(43) Veröffentlichungstag der Anmeldung: 19.01.83 Patentblatt 83/3

(84) Benannte Vertragsstaaten: DE FR GB

(71) Anmelder: HOECHST AKTIENGESELLSCHAFT
Postfach 80 03 20
D-6230 Frankfurt Main 80(DE)

(72) Erfinder: Lal, Bansi, Dr.
Advani Apartments 30 Mulund (West)
Bombay-400 080(IN)

(72) Erfinder: D'Sa, Adolf
Laxmi Apartments 17 Station Road Vikhroli (West)
Bombay-400 079(IN)

(72) Erfinder: Nomanbhai Dohadwalla, Alihussein, Dr.
Noman Mansion 139C Cumballa Hill
Bombay-400 036(IN)

(72) Erfinder: de Souza, Noel John, Dr.
Avanti Central Avenue 702 Santa Cruz
Bombay-400 054(IN)

(72) Erfinder: Dornauer, Horst, Dr.
Am Kirchplatz 14
D-6233 Kelkheim (Taunus)(DE)

(54) **Triazino-(2,1-a)isochinolinderivate, Verfahren zu ihrer Herstellung und ihre Verwendung als Arzneimittel.**

(57) Die vorliegende Erfindung betrifft neue Triazino-(2,1-a)-isochinolinderivate sowie ein Verfahren zu ihrer Herstellung. Die erfindungsgemäßen Verbindungen besitzen wertvolle pharmakologische Eigenschaften und können daher als Arzneimittel verwendet werden.

EP 0 069 953 A1

Triazino-(2,1-a)isochinolinderivate, Verfahren zu ihrer Herstellung und ihre Verwendung als Arzneimittel.

Die Erfindung betrifft eine neue Klasse von Triazino-(2,1-a)-isochinolinderivaten und ein Verfahren zur Herstellung der erfindungsgemäßen Verbindungen. Die Triazino-(2,1-a)-isochinolinderivate der vorliegenden Erfindung besitzen wertvolle pharmakologische Eigenschaften, zum Beispiel blutdrucksenkende Eigenschaften, nachweisbar an Katzen und Hunden.

Gegenstand der Erfindung sind also Triazino-(2,1-a)-isochinolinderivate mit einem neuen heterocyclischen Ringsystem der Formel I

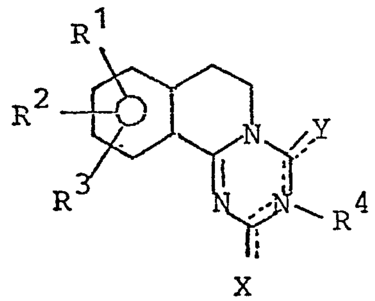

worin $R^1$, $R^2$ und $R^3$ gleich oder verschieden sein können und Wasserstoff, niederes Alkoxy, Acyloxy oder Halogen sein können, wobei zwei benachbarte Reste $R^1$, $R^2$ oder $R^3$ zusammen eine Alkylendioxygruppe, vorzugsweise eine Methylendioxy- oder Äthylendioxygruppe, bedeuten können; $R^4$ ein Elektronenpaar, Wasserstoff oder niederes Alkyl bedeutet;
X Halogen, ein Sauerstoff- oder Schwefelatom, Imino, das mit niederem Alkyl oder Aryl substituiert sein kann, Alkylthio, Alkoxy oder $NR^5R^6$, worin $R^5$ und $R^6$ jeweils Wasserstoff, niederes Alkoxy, Amino, niederes Alkylamino, niederes Dialkylamino, Acylamino, niederes Alkoxycarbonylamino, einen stickstoffhaltigen heterocyclischen Rest, niederes Alkyl, $C_3$-$C_6$-Cycloalkyl, niederes Dialkylamino-

alkyl, Aralkyl oder heterocyclisch substituiertes niederes Alkyl oder Aryl sein können oder worin $R^5$ und $R^6$ zusammen mit dem Stickstoffatom, an das sie gebunden sind, einen gegebenenfalls substituierten Stickstoffheterocyclus, der ein weiteres Stickstoff- oder Sauerstoffatom enthalten kann, sein können,
Y ein Sauerstoff- oder Schwefelatom, niederes Alkylthio oder $NR^7R^8$, worin $R^7$ und $R^8$ die für $R^5$ und $R^6$ angegebenen Bedeutungen haben, bedeutet,
sowie deren Säureadditionssalze.

Unter "niedere Gruppen" in obigem Zusammenhang versteht man Gruppen mit 1 bis 6 Kohlenstoffatomen.

Bevorzugte niedere Alkoxygruppen für $R^1$, $R^2$, $R^3$, $R^5$, $R^6$, $R^7$ oder $R^8$ sind z.B. solche mit bis zu 3 Kohlenstoffatomen.

Geeignete Acyloxyreste für $R^1$, $R^2$ oder $R^3$ sind beispielsweise solche, in denen die Acylgruppe eine geradkettige oder verzweigte Alkanoylgruppe mit 1 - 6 Kohlenstoffatomen bedeutet, z.B. die Acetylgruppe, oder eine Aroylgruppe, insbesondere die Benzoylgruppe mit gegebenenfalls ein- bis dreifach durch Halogen, Nitro, Hydroxy, $C_1$-$C_3$-Alkoxy- und $C_1$-$C_3$-Alkylgruppen substituiertem Phenylkern.

Falls $R^1$, $R^2$, $R^3$ oder X ein Halogenatom bedeuten, sei als bevorzugter Rest z.B. Chlor erwähnt.

Als Alkylreste für X, Y, $R^4$, $R^5$, $R^6$, $R^7$ oder $R^8$ eignen sich solche mit höchstens 6 Kohlenstoffatomen, wie z.B. Methyl,Äthyl, n-Propyl, Isopropyl, Isobutyl oder tert.-Butyl.

Als Alkylamino- oder Dialkylaminoreste für $R^5$, $R^6$, $R^7$ oder $R^8$ eignen sich besonders solche mit Alkylgruppen mit höchstens 3 Kohlenstoffatomen, z.B. Methylamino- oder Dimethylaminogruppen.

Als Acylaminorest für $R^5$, $R^6$, $R^7$ oder $R^8$ eignet sich insbesondere der Äthoxycarbonylaminorest.

Als geeignete Cycloalkylreste für $R^5$, $R^6$, $R^7$ oder $R^8$ seien solche mit höchstens 6 Kohlenstoffatomen erwähnt, wie z.B. Cyclohexyl.

Als ein substituierter Alkylrest für $R^5$, $R^6$, $R^7$ oder $R^8$ eignet sich ein ein- oder zweifach durch Di-($C_1$-$C_4$)-alkylaminogruppen substituierter Rest mit bis zu 6 Kohlenstoffatomen.

Beispiele für Aralkylreste für $R^5$, $R^6$, $R^7$ oder $R^8$ sind solche mit höchstens 8 Kohlenstoffatomen, in welchen der Arylrest ein ein- oder mehrfach, insbesondere ein ein-, zwei- oder dreifach durch Halogen, Nitro, Hydroxy, $C_1$-$C_3$-Alkoxy oder $C_1$-$C_3$-Alkyl substituierter Phenylrest sein kann.

Als geeignete stickstoffhaltige heterocyclische Reste seien beispielsweise Piperidino, Morpholino oder Piperazino erwähnt, die durch $C_1$-$C_3$-Alkyl, Aralkyl, $C_1$-$C_3$-Alkoxy, Aryl oder Stickstoffheterocyclen mit der oben angegebenen Bedeutung substituiert sein können.

Geeignete Arylreste sind unsubstituierte oder ein-, zwei- oder dreifach durch Halogen, Hydroxy, Nitro, $C_1$-$C_3$-Alkoxy oder $C_1$-$C_3$-Alkyl substituierte Phenylreste.

Als Salze der erfindungsgemäßen Triazino-(2,1-a)-isochinolinderivate seien beispielsweise solche von anorganischen oder organischen Säuren erwähnt, wie Hydrochloride, Hydrobromide, Sulfate, Phosphate, Acetate, Oxalate, Tartrate, Citrate, Maleate oder Fumarate.

Bevorzugte Substituenten sind:

für $R^1$ oder $R^2$: $C_1$-$C_3$-Alkoxy;
für $R^3$: Wasserstoff;
für $R^4$: ein Elektronenpaar, Wasserstoff oder $C_1$-$C_6$-Alkyl;
für X : Sauerstoff, eine Niederalkylimino oder Aryliminogruppe oder die Gruppe $NR^5R^6$, wobei $R^5$ und $R^6$ vorzugsweise für Wasserstoff, stickstoffhaltige heterocyclische Reste, niederes Alkyl oder Aryl stehen und wobei $R^5$ und $R^6$ zusammen mit dem Stickstoffatom an das sie gebunden sind, einen gegebenenfalls substituierten Stickstoffheterocyclus, der ein weiteres Heteroatom enthalten kann, bedeuten;
für Y : Sauerstoff, Schwefel, Alkylthio oder die Gruppe $NR^7R^8$, wobei $R^7$ und $R^8$ vorzugsweise Wasserstoff, einen Stickstoffheterocyclus, niederes Acylamino, niederes Alkyl oder Aryl bedeuten oder worin $R^7$ und $R^8$ zusammen mit dem Stickstoffatom, an das sie gebunden sind, einen gegebenenfalls substituierten Stickstoffheterocyclus bedeuten, der ein weiteres Heteroatom enthalten kann.

Besonders bevorzugte Verbindungen gemäß vorliegender Erfindung sind:

9,10-Dimethoxy-2-tert.-butylamino-6,7-dihydro-4H-triazino-(2,1-a)-isochinolin-4-on-hydrochlorid,
9,10-Dimethoxy-2-(2,4-dichloranilino)-6,7-dihydro-4H-triazino-(2,1-a)-isochinolin-4-on-hydrochlorid,
9,10-Dimethoxy-2-mesitylamino-6,7-dihydro-4H-triazino-(2,1-a)-isochinolin-4-on-hydrochlorid-monohydrat,
9,10-Dimethoxy-2-morpholino-6,7-dihydro-4H-triazino-(2,1-a)-isochinolin-4-on-hydrochlorid,
9,10-Dimethoxy-2-mesitylimino-4-(4-methylanilino)-6,7-dihydro-2H-triazino-(2,1-a)-isochinolin-hydrojodid,
9,10-Dimethoxy-2-mesitylimino-4-amino-6,7-dihydro-2H-triazino-(2,1-a)-isochinolin-hydrojodid-monohydrat,
9,10-Dimethoxy-2-mesitylimino-4-(2,4-dimethylanilino)-6,7-dihydro-2H-triazino-(2,1-a)-isochinolin-hydrojodid-

monohydrat.

Einige neue Triazino-(2,1-a)-isochinolinderivate, sowie ihre Salze und Schmelzpunkte sind aus der folgenden Tabelle ersichtlich.

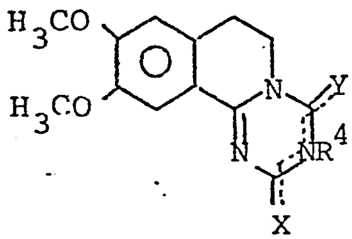

| $R^4$ | X | Y | Salz | Schmelzpunkt (°C) |
|---|---|---|---|---|
| H | O | O | | 292 - 93 |
| : | $NH_2$ | O | - | 283 - 84 |
| : | NH-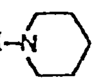 | O | $HCl.0.5H_2O$ | 275 - 78 |
| : | $HN(CH_2)_3CH_3$ | O | $HCl.H_2O$ | 248 - 50 |
| : | $HNCHMe_2$ | O | $HCl.H_2O$ | 228 - 31 |
| : | $HNCH_2CHMe_2$ | O | $HCl.H_2O$ | 215 - 18 |
| : | $HNCMe_3$ | O | HCl | 243 - 45 |
| : | HN-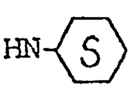 | O | HCl | 238 - 39 |

| $R^4$ | X | Y | Salz | Schmelzpunkt (°C) |
|---|---|---|---|---|
| : | $HN(CH_2)_3NMe_2$ | O | $2HCl\cdot H_2O$ | 238 – 41 |
| : | 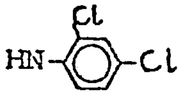 | O | HCl | 222 – 24 |
| : | 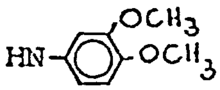 | O | HCl | 270 – 71 |
| : | 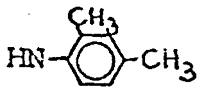 | O | HCl | 222 – 24 |
| : | 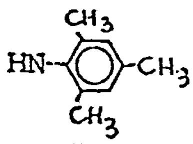 | O | $HCl\cdot H_2O$ | 240 – 42 |
| : | 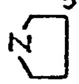 | O | HCl | 255 – 56 |
| : | 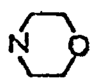 | O | HCl | 252 – 53 |
| : | 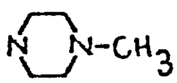 | O | $HCl\cdot 0.5H_2O$ | 275 – 78 |
| : | $NCHMe_2$ | S | – | 253 – 54 |
| : | $NCHMe_2$ | $SC_2H_5$ | HI | 208 – 11 |
| : | $NCHMe_2$ | $NH_2$ | $CH_3SO_3H\cdot H_2O$ | 204 – 6 |
| : | $NCHMe_2$ | 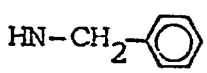 | HI | 240 – 41 |
| : | $NCHMe_2$ | 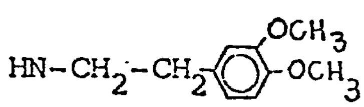 | HI | 220 – 22 |
| : | $NCHMe_2$ | $HNCH_2CHMe_2$ | HI | 247 – 49 |
| : | $NCHMe_2$ | 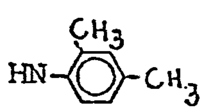 | HI | 246 – 48 |
| : | $NCHMe_2$ | 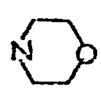 | HI | 214 – 15 |
| : | 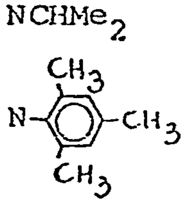 | $NH_2$ | $HI-H_2O$ | 261 – 63 |

| $R^4$ | X | Y | Salz | Schmp. (°C) |
|---|---|---|---|---|
| : | 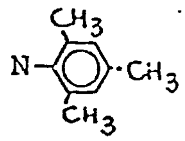 | $NH-NHCO_2Et$ | $0.5H_2O$ | 276 - 78 |
| : | 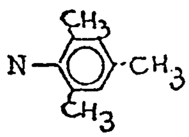 | $HNCH_2CHMe_2$ | $CH_3SO_3H.2H_2O$ | 186 - 87 |
| : | 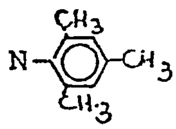 | $HN(CH_2)_9CH_3$ | $CH_3SO_3H$ | 122 - 25 |
| : | 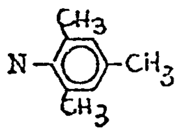 | 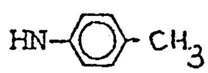 | HI | 258 - 60 |
| : | 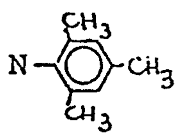 | 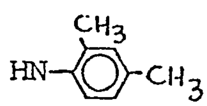 | $HI.H_2O$ | 252 - 55 |
| : | 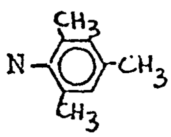 | 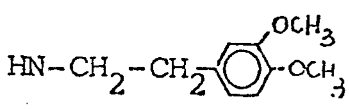 | $CH_3SO_3H.2H_2O$ | 219 - 20 |
| : | 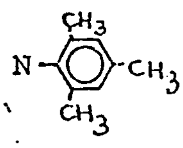 | 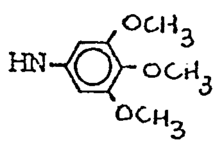 | $CH_3SO_3H$ | 285 - 88 |

Gegenstand der vorliegenden Erfindung ist weiterhin ein Verfahren zur Herstellung der erfindungsgemäßen Triazino-(2,1-a)-isochinolinderivate, das dadurch gekennzeichnet ist, daß

a) Verbindungen der Formel I, worin $R^4$ Wasserstoff und X und Y je Sauerstoff bedeuten, dargestellt durch die Formal Ia

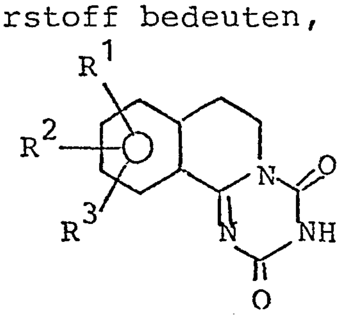

(Ia)

durch Reaktion einer Verbindung der Formel II

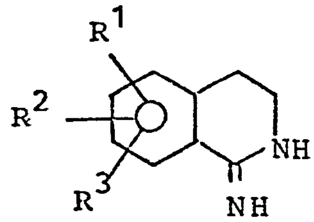

. HX (II)

worin $R^1$, $R^2$ und $R^3$ die oben angegebene Bedeutung haben und X für ein Halogenatom, z.B. ein Chlor-, Brom- oder Jodatom steht,

mit einer Verbindung der Formel III

$$O=C=N-\overset{O}{\overset{\|}{C}}-R^6 \qquad \text{(III)}$$

worin $R^6$ eine abspaltbare Schutzgruppe bedeutet, wie z.B. Ethoxygruppe, in Gegenwart einer Base wie z.B. Natriumhydrid und eines Lösungsmittels z.B. ein aromatischer Kohlenwasserstoff wie Benzol, bei Temperaturen von Zimmertemperatur bis zum Siedepunkt des jeweiligen Lösungsmittels, hergestellt werden, wobei die Verbindungen der Formel II nach bekannten Verfahren hergestellt werden können (siehe S. African Patent 6901, 552 (1969), cf. C.A. 72, 111309 p (1970); C.A. 58, 503 (1963);

oder daß

b) Verbindungen der Formel I, worin $R^4$ ein Elektronenpaar, X die Gruppe $NR^5R^6$ und Y ein Sauerstoffatom bedeuten, dargestellt durch die Formel Ib

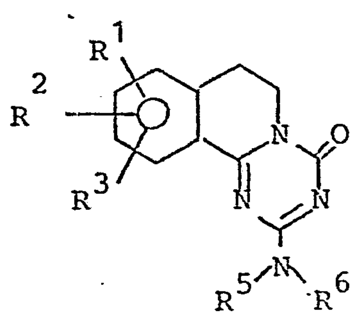

(Ib)

durch Umsetzung von Verbindungen der Formel Ia mit einem

Halogenid wie z.B. Phosphoroxytrichlorid und nachfolgende Reaktion mit Verbindungen der Formel IV

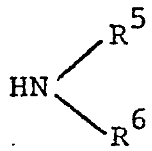

(IV)

worin $R^5$ und $R^6$ die oben angegebene Bedeutung haben, gegebenenfalls in Gegenwart einer Base oder eines Säurefängers wie beispielsweise eine Verbindung der Formel $HN<^{R^5}_{R^6}$ , die im Überschuß eingesetzt wird, oder eines Alkalihydrids wie beispielsweise Natriumhydrid oder eines tertiären Amins wie beispielsweise Triethylamin oder eines Säureakzeptors wie beispielsweise Diazobicyclononen, hergestellt werden, wobei die Reaktion in Gegenwart eines polaren Lösungsmittels wie Dimethylformamid, Dimethylsulfoxid, von halogenierten Kohlenwasserstoffen wie Chloroform oder von Alkanolen wie Butanol, von aprotischen Lösungsmitteln wie hochsiedende Ether, beispielsweise Diethylenglycoldimethylether durchgeführt werden kann und durch Erhitzen bis zum Siedepunkt des jeweiligen Lösungsmittels, vervollständigt oder beschleunigt werden kann,

oder daß

c) Verbindungen der Formel I, worin $R^4$ ein Elektronenpaar, X eine Gruppe der Formel $NR^5R^6$ und Y ein Sauerstoffatom bedeuten, dargestellt durch die Formel Ic

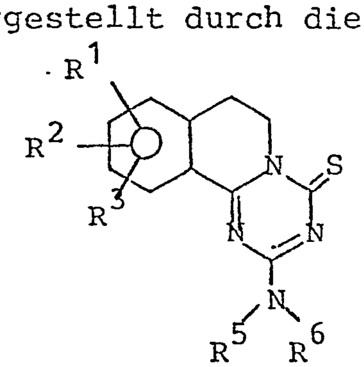

(Ic)

durch Umsetzung einer Verbindung der Formel Ib mit einem Sulphid wie beispielsweise Phosphorpentasulfid oder 2,4-Bis-(4-methoxyphenyl)-1,3,2,4-dithiadiphosphetan-2,4-disulfid /S. Scheibye, B.S. Pedersen und S.-O. Lawesson, Bull. Soc. Chem. Belg. 87, 229 (1978)/ in Gegenwart eines Lösungsmittels,

beispielsweise eines aromatischen Kohlenwasserstoffes, wie Benzol oder eines aprotischen polaren Lösungsmittels wie beispielsweise Hexamethylphosphortriamid oder Diphenyl, bei einer Temperatur von 50 - 150°C, hergestellt werden oder daß d) Verbindungen der Formel I, worin $R^4$ ein Elektronenpaar, X eine Gruppe der Formel $NR^5R^6$, worin $R^6$ ein Elektronenpaar darstellt, Y SAlk und Alk eine $C_1$-$C_4$-Alkylgruppe wie Ethyl, bedeuten, dargestellt durch die Formel Id

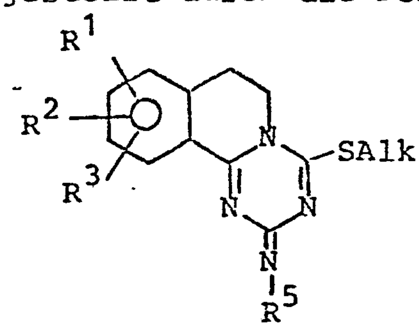

(Id)

durch Umsetzung einer Verbindung der Formel Ic, worin $R^6$ Wasserstoff bedeutet, mit einem Alkylhalogenid wie z.B. Ethyljodid, in Gegenwart eines polaren Lösungsmittels wie z.B. Dimethylformamid, von halogenierten Kohlenwasserstoffen wie z.B. Chloroform oder eines aprotischen Lösungsmittels wie z.B. Tetrahydrofuran, hergestellt werden, wobei die Reaktion durch Hitzeanwendung, z.B. durch Erhitzen bis zum Siedepunkt des jeweiligen Lösungsmittels, vervollständigt oder beschleunigt werden kann, oder daß e) Verbindungen der Formel I, worin $R^4$ ein Elektronenpaar, X eine Gruppe der Formel $NR^5R^6$, worin $R^6$ für ein Elektronenpaar steht, Y eine Gruppe der Formel $N<^{R^7}_{R^8}$, worin $R^7$ und $R^8$ die oben angegebenen Bedeutungen besitzen, bedeuten, dargestellt durch die Formel Ie

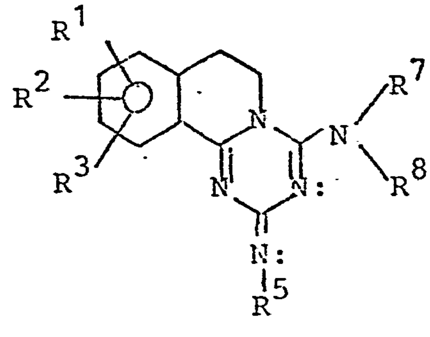

(Ie)

durch Umsetzung einer Verbindung der Formel Id mit einer Verbindung der Formel V

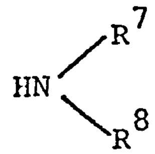

(V)

worin $R^7$ und $R^8$ die oben angegebenen Bedeutungen besitzen, gegebenenfalls in Gegenwart einer Base oder eines Säurefängers, beispielsweise einer Verbindung der Formel $NHR^7R^8$, die im Überschuß eingesetzt wird, oder eines Alkalihydrids wie z.B. Natriumhydrid, eines tertiären Amins wie z.B. Triethylamin oder eines Säureakzeptors, wie z.B. Diazobicyclononen, hergestellt werden, wobei die Reaktion in Gegenwart eines polaren Lösungsmittels wie z.B. Dimethylformamid, Dimethylsulfoxid, von halogenierten Kohlenwasserstoffen wie z.B. Chloroform, von Alkanolen wie z.B. Butanol oder von aprotischen Lösungsmitteln, wie hochsiedende Ether, z.B. Diethylenglycoldimethylether, durchgeführt werden kann und durch Erhitzen bis zum Siedepunkt des jeweiligen Lösungsmittels, vervollständigt oder beschleunigt werden kann.

Die erhaltene erfindungsgemäße Verbindung der Formel I kann gegebenenfalls in ihr Säureadditionssalz mit einer der oben als Beispiel angeführten Säuren überführt werden.

Die erfindungsgemäßen Triazino-(2,1-a)-isochinolinderivate besitzen blutdrucksenkende Wirkung, wie durch Tierversuche an Hunden und Katzen festgestellt worden ist,und eig nen sich deshalb zur Behandlung des Bluthochdrucks in der Human- und Tiermedizin.

Aufgrund der blutdrucksenkenden Wirkung sind die neuen Wirkstoffe für die Behandlung und Prophylaxe von Herz-Kreislaufkrankheiten wie z.B. essentielle und maligne Hypertonie, Herzinsuffizienz, Angina pectoris und Störungen des peripheren Kreislaufs geeignet. Die Wirkstoffe können auch in Verbindung

mit anderen pharmakologisch wirksamen Substanzen eingesetzt werden z.B. mit Diuretika, Antiarrhytmika, ß-Blockern, Beruhigungsmitteln, herzgefäßerweiternden Mitteln, Hypolipidemika usw.

Die erfindungsgemäßen Wirkstoffe können peroral, parenteral (intramuskulär, intravenös, subkutan), rektal, als Aerosol verabreicht oder lokal angewandt werden.

An Säugetieren einschließlich Menschen werden die folgenden Dosen verwendet:

Zur Senkung des Blutdrucks: Tagesdosis 0,1 - 200 mg; Einheitsdosis 0,1 - 25 mg.

Die neuen Verbindungen können entweder allein oder mit pharmakologisch verträglichen Trägern vermischt angewandt werden. Für eine orale Anwendungsform werden die aktiven Verbindungen mit den dafür üblichen Substanzen vermischt und durch übliche Methoden in geeignete Darreichungsformen gebracht, wie Tabletten, Steckkapseln, wäßrige, alkoholische oder ölige Suspensionen oder wäßrige, alkoholische oder ölige Lösungen. Als inerte Träger können z.B. Magnesiumcarbonat Milchzucker oder Maisstärke unter Zusatz anderer Stoffe wie z.B. Magnesiumstearat verwendet werden. Dabei kann die Zubereitung sowohl als Trocken- oder Feuchtgranulat erfolgen. Als ölige Trägerstoffe oder Lösungsmittel kommen besonders pflanzliche und tierische Öle in Betracht wie z.B. Sonnenblumenöl oder Lebertran.

Eine z.B. für die Notfalltherapie wichtige Anwendungsform liegt in der intravenösen Applikation. Zu diesem Zweck werden die aktiven Verbindungen oder deren physiologisch verträgliche Salze, soweit sie eine ausreichende Löslichkeit besitzen, mit den dafür üblichen Hilfsstoffen, die z.B. lösungsvermittelnde oder puffernde Eigenschaften haben können, in Lösung gebracht.

Physiologisch verträgliche Salze werden z.B. mit folgenden Säuren gebildet: Chlor-, Brom- oder Jodwasserstoffsäure, Phosphorsäure, Schwefelsäure, Methylschwefelsäure, Amidosulfonsäure, Salpetersäure, Weinsäure, Milchsäure, Malonsäure, Fumarsäure, Oxalsäure, Zitronensäure, Äpfelsäure, Schleimsäure, Benzoesäure, Salicylsäure, Acetursäure, Embonsäure, Naphthalin-1,5-disulfonsäure, Ascorbinsäure, Phenylessigsäure, p-Aminosalicylsäure, Hydroxyäthansulfonsäure, Benzolsulfonsäure oder synthetische Harze, die saure Gruppen enthalten, z.B. solche mit Ionenaustauscherwirkung.

Als Lösungsmittel für eine intravenöse Applikation kommen z.B. in Frage: Wasser, physiologische Kochsalzlösung oder verdünnte Alkohole wie z.B. Ethanol, Propandiol oder Glycerin, daneben auch Zuckerlösungen wie z.B. Glukose- oder Mannitlösungen, oder auch eine Mischung aus den verschiedenen genannten Lösungsmitteln.

Die folgenden Beispiele erläutern die Erfindung:

Beispiel 1

9,10-Dimethoxy-3,4,6,7-tetrahydro-2H-triazino-(2,1-a)-isochinolin-2,4-dion

Eine Suspension aus 9,00 g Natriumhydrid in 250 ml trockenem Benzol wird mit 40 g 6,7-Dimethoxy-1-imino-1,2,3,4-tetrahydroisochinolin-hydrojodid versetzt und 3 Stunden unter Rückfluß erhitzt. Nach Zugabe von 30,0 ml Ethoxycarbonylisocyanat in 200 ml Benzol wird die erhaltene Mischung 16 Stunden lang unter Rückfluß erhitzt. Das überschüssige Natriumhydrid wird mit Methanol zersetzt und das Reaktionsgemisch mit 500 ml Wasser verdünnt. Die wäßrige Phase wird abgetrennt und mit verdünnter Salzsäure angesäuert, wobei man einen Feststoff erhält, der filtriert, mit Wasser gewaschen, getrocknet und aus Methanol umkristallisiert wird. Ausbeute: 26,0 g. Schmelzpunkt: 292 - 293°C.

Beispiel 2

Allgemeines Verfahren zur Herstellung der Verbindungen der Formel I aus Verbindungen der Formel V

Ein Gemisch aus einer Verbindung der Formel V und Phosphoroxytrichlorid (10 - 20 Mol) wird 3 Stunden unter Rückfluß erhitzt. Das überschüssige Phosphoroxytrichlorid wird dann unter erhöhtem Druck abdestilliert und der Rückstand mit einem entsprechenden, im Überschuß (5 - 10 Mol) eingesetzten Amin der Formel $HN<\begin{matrix}R^5\\R^6\end{matrix}$ in Gegenwart einer Base, vorzugsweise des im Überschuß eingesetzten Amins als Reagenz, oder eines Säurefängers und vorzugsweise in Gegenwart eines entsprechenden, wie oben aufgeführten Lösungsmittels erhitzt. Das Reaktionsgemisch kann 2 bis 20 Stunden bei Rückflußtemperatur erhitzt werden. Der nach Verdampfen des Lösungsmittels bei verringertem Druck erhaltene Rückstand wird mit Wasser versetzt und mit einem organischen Lösungsmittel extrahiert. Der erhaltene Extrakt kann dann entsprechend gewaschen, über wasserfreiem Natriumsulfat stehen gelassen und zur Trockne eingedampft werden. Der dabei erhaltene Rückstand kann chromatographisch gereinigt und zur gewünschten Verbindung umkristallisiert werden, wobei letztere gegebenenfalls in ihr Säureadditionssalz überführt werden kann.

Beispiel 3

9,10-Dimethoxy-2-tert.-butylamino-6,7-dihydro-4H-triazino-(2,1-a)-isochinolin-4-on-hydrochlorid

Ein Gemisch aus 1,0 g 9,10-Dimethoxy-3,4,6,7-tetrahydro-2H-triazino-(2,1-a)-isochinolin-2,4-dion und 10 ml Phosphoroxytrichlorid wird 3 Stunden unter Rückfluß erhitzt und überschüssiges Phosphoroxytrichlorid wird unter vermindertem Druck abdestilliert.

Der Rückstand wird dann in 30 ml Chloroform gelöst, mit 5 ml

tertiärem Butylamin versetzt und 20 Stunden unter Rückfluß erhitzt. Überschüssiges Amin wird unter vermindertem Druck abdestilliert. Der Rückstand wird mit Chloroform extrahiert. Das erhaltene Extrakt wird nacheinander mit 10 %igem Natriumhydroxid und Wasser gewaschen, über wasserfreiem Natriumsulfat getrocknet und unter vermindertem Druck eingedampft. Der erhaltene Rückstand wird durch Reaktion mit ätherischer Salzsäure in Ethanol in sein Hydrochlorid überführt, das dann aus Methanol/Wasser umkristallisiert wird. Ausbeute: 0,8 g. Schmelzpunkt: 243 - 245°C.

Beispiel 4

9,10-Dimethoxy-2-(2,4-dichloranilino)-6,7-dihydro-4H-triazino-(2,1-a)-isochinolin-4-on-hydrochlorid

Man verfährt im wesentlichen wie in Beispiel 3 beschrieben, jedoch unter Verwendung von 2,4-Dichloranilin anstelle von tert.-Butylamin. Vor Überführung in sein Hydrochlorid, wird das Verfahrensprodukt durch Überleiten über eine Silikagelsäule mit Chloroform als Eluiermittel gereinigt. Ausbeute an Hydrochlorid: 47 %. Schmelzpunkt: 222 - 224°C.

Beispiel 5

9,10-Dimethoxy-2-mesitylamino-6,7-dihydro-4H-triazino-(2,1-a)-isochinolin-4-on-hydrochlorid

Man verfährt im wesentlichen wie in Beispiel 3 beschrieben, jedoch unter Verwendung von Mesitylamin anstelle von tert.-Butylamin. Ausbeute an Hydrochlorid: 55 %. Schmelzpunkt: 240 - 242°C.

Beispiel 6

9,10-Dimethoxy-2-morpholino-6,7-dihydro-4H-triazino-(2,1-a)-isochinolin-4-on-hydrochlorid

Man verfährt im wesentlichen wie in Beispiel 3 beschrieben,

jedoch unter Verwendung von Morpholin anstelle von tert.-Butylamin. Ausbeute an Hydrochlorid: 73 %. Schmelzpunkt: 252 - 253°C.

Beispiel 7

9,10-Dimethoxy-2-mesitylamino-6,7-dihydro-2H-triazino-(2,1-a)-isochinolin-4-thion

Ein Gemisch aus 14,0 g 9,10-Dimethoxy-2-mesitylamino-6,7-dihydro-4H-triazino-(2,1-a)-isochinolin-4-on und 12,0 g 2,4-Bis-(4-methoxyphenyl)-1,3,2,4-dithiadiphosphetan-2,4-disulfid in 350 ml Dioxan wird 16 Stunden auf 95 - 100°C erhitzt, überschüssiges Lösungsmittel wird unter verringertem Druck verdampfen gelassen und der Rückstand wird mit Wasser versetzt und mit Chloroform extrahiert.

Der Extrakt wird nacheinander mit 10 %igem Natriumhydroxid und mit Wasser gewaschen, die organische Schicht über wasserfreiem Natriumsulfat stehen gelassen und zur Trockne eingedampft. Der erhaltene Rückstand wird durch Überleiten über eine Silikagelsäule unter Verwendung von Chloroform als Eluiermittel gereinigt. Nach Abdampfen des Lösungsmittels erhält man das entsprechende 4-Thion. Ausbeute: 10,0 g. Schmelzpunkt 253 - 254°C.

Beispiel 8

9,10-Dimethoxy-2-mesitylimino-4-ethylthio-6,7-dihydro-2H-triazino-(2,1-a)-isochinolin-hydrojodid

Eine wie in Beispiel 7 hergestellte Lösung aus 10,0 g 9,10-Dimethoxy-2-mesitylamino-6,7-dihydro-4H-triazino-(2,1-a)-isochinolin-4-thion in 300 ml Tetrahydrofuran wird mit 25 ml Ethyljodid versetzt und das erhaltene Reaktionsgemisch wird 2 Stunden erhitzt. Der erhaltene Feststoff wird filtriert, wobei das 9,10-Dimethoxy-2-mesitylimino-4-ethylthio-6,7-dihydro-2H-triazino-(2,1-a)-isochinolin-

hydrojodid in einer Ausbeute von 11,50 g anfällt. Schmelzpunkt: 208 - 211!C.

Beispiel 9

9,10-Dimethoxy-2-mesitylimino-4-(4-methylanilino)-6,7-dihydro-2H-triazino-(2,1-a)-isochinolin-hydrojodid

Ein Gemisch aus 2,5 g der in Beispiel 8 erhaltenen Verbindung und aus 2,5 g 4-Methylanilin wird 4 Stunden bei 100°C erhitzt. Das erhaltene Reaktionsprodukt wird abgekühlt und mit überschüssigem Diethylether verrieben, wobei ein Feststoff anfällt, der filtriert und durch Umkristallisieren aus Methanol/Diethylether gereinigt wird. Ausbeute: 1,8 g. Schmelzpunkt: 258 - 260°C.

Beispiel 10

9,10-Dimethoxy-2-mesitylimino-4-amino-6,7-dihydro-2H-triazino-(2,1-a)-isochinolin-monohydrat

2,5 g der in Beispiel 8 erhaltenen Verbindung werden mit 25 ml einer gesättigten Lösung aus Ammoniak in Ethanol versetzt und die erhaltene Lösung 2 Stunden bei Zimmertemperatur gerührt. Die Lösung wird im Vakuum stark eingedampft und mit Diethylether verdünnt, wobei man einen Feststoff erhält, der aus einem Methanol/Ether-Gemisch umkristallisiert wird. Ausbeute: 2,0 g. Schmelzpunkt 261 - 263°C.

Beispiel 11

9,10-Dimethoxy-2-mesitylimino-4-(2,4-dimethylanilino)-6,7-dihydro-2H-triazino-(2,1-a)-isochinolin-hydrojodid

Ein Gemisch aus 2,0 g der in Beispiel 8 erhaltenen Verbindung und 5 ml 2,4-Dimethylanilin wird 4 Stunden bie 100°C erhitzt. Die erhaltene Mischung wird abgekühlt und mit Diethylether verrieben, wobei ein Feststoff anfällt, der durch Umkristallisation aus einem Methanol/Diethylether-Gemisch

gereinigt wird. Ausbeute: 1,7 g. Schmelzpunkt: 252 - 255°C.

Patentansprüche:

1. Verbindungen der Formel I

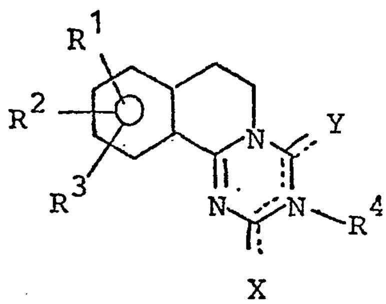

(I)

worin $R^1$, $R^2$ und $R^3$ gleich oder verschieden sein können und Wasserstoff, niederes Alkoxy, Acyloxy oder Halogen sein können, wobei zwei benachbarte Reste $R^1$, $R^2$ oder $R^3$ zusammen eine Alkylendioxygruppe, vorzugsweise eine Methylendioxy- oder Ethylendioxygruppe bedeuten können; $R^4$ ein Elektronenpaar, Wasserstoff oder niederes Alkyl bedeutet;
X Halogen, ein Sauerstoff- oder Schwefelatom, eine Iminogruppe, die mit nieder-Alkyl oder Aryl substituiert sein kann, Alkylthio, Alkoxy oder $NR^5R^6$, worin $R^5$ und $R^6$ jeweils Wasserstoff, nieders Alkoxy, Amino, niederes Alkylamino, niederes Dialkylamino, Acylamino, niederes Alkoxycarbonylamino, einen stickstoffhaltigen heterocyclischen Rest, niederes Alkyl, $C_3$-$C_6$-Cycloalkyl, niederes Dialkylaminoalkyl, Aralkyl oder heterocyclisch substituiertes niederes Alkyl oder Aryl sein können oder worin $R^5$ und $R^6$ zusammen mit dem Stickstoffatom, an das sie gebunden sind, einen gegebenenfalls substituierten Stickstoffheterocyclus, der ein weiteres Stickstoff- oder Sauerstoffatom enthalten kann, sein können,
Y ein Sauerstoff- oder Schwefelatom, niederes Alkylthio oder $NR^7R^8$, worin $R^7$ und $R^8$ die für $R^5$ und $R^6$ angegebenen Bedeutungen haben, bedeutet,
sowie deren Säureadditionssalze.

2. Verbindungen der Formel I gemäß Anspruch 1, worin $R^1$ und/oder $R^2$ $C_1$-$C_3$-Alkoxy, $R^3$ Wasserstoff und $R^4$ ein

Elektronenpaar, Wasserstoff oder $C_1$-$C_6$-Alkyl bedeuten, X Sauerstoff, eine Iminogruppe, die mit niederem Alkyl oder Aryl substituiert sein kann, oder die Gruppe $NR^5R^6$, wobei $R^5$ und $R^6$ für Wasserstoff, stickstoffhaltige heterocyclische Reste, $C_1$-$C_6$-Alkyl oder Aryl stehen und wobei $R^5$ und $R^6$ zusammen mit dem Stickstoffstom, an das sie gebunden sind, einen gegebenenfalls substituierten Stickstoffheterocyclus, der ein weiteres Heteroatom enthalten kann, bedeuten, und Y Sauerstoff, Schwefel, $C_1$-$C_6$-Alkylthio oder die Gruppe $NR^7R^8$, wobei $R^7$ und $R^8$ Wasserstoff, einen Stickstoffheterocyclus, $C_1$-$C_6$-Acylamino, $C_1$-$C_6$-Alkyl oder Aryl bedeuten oder worin $R^7$ und $R^8$ zusammen mit dem Stickstoffatom, an das sie gebunden sind, einen gegebenenfalls substituierten Stickstoffheterocyclus bedeuten, der ein weiteres Heteroatom enthalten kann.

3. Verbindungen der Formel I gemäß Anspruch 1 und 2, worin Y Sauerstoff und X die Gruppe $NR^5R^6$ bedeuten, wobei entweder $R^5$ Wasserstoff und $R^6$ Wasserstoff, $C_1$-$C_6$-Alkyl, ein- bis dreifach mit Halogen, $C_1$-$C_3$-Alkyl oder $C_1$-$C_3$-Alkoxy substituiertes Phenyl oder Piperidino bedeuten, oder $R^5$ und $R^6$ zusammen einen unsubstituierten oder substituierten Pyrrolidino-, Piperazino- oder Morpholino-Rest darstellen.

4. Verbindungen der Formel I gemäß Anspruch 1, worin X eine Iminogruppe darstellt, die mit $C_1$-$C_6$-Alkyl oder Aryl substituiert sein kann und Y Schwefel, $C_1$-$C_6$-Alkylthio oder die Gruppe $NR^7R^8$ bedeutet, wobei entweder $R^7$ Wasserstoff und $R^8$ Wasserstoff, $C_1$-$C_6$-Alkyl, $C_2$-$C_6$-Alkoxycarbonylamino, oder ein- bis dreifach mit Halogen, $C_1$-$C_3$-Alkyl oder $C_1$-$C_3$-Alkoxy substituiertes Phenyl bedeuten, oder $R^7$ und $R^8$ zusammen einen unsubstituierten Pyrrolidino, Piperazino- oder Morpholino-Rest darstellen.

5. 9,10-Dimethoxy-2-tert.-butylamino-6,7-dihydro-4H-triazino-(2,1-a)-isochinolin-4-on-hydrochlorid.

6. 9,10-Dimethoxy-2-(2,4-dichloranilino)-6,7-dihydro-4H-triazino-(2,1-a)-isochinolin-4-on-hydrochlorid.

7. 9,10-Dimethoxy-2-mesitylamino-6,7-dihydro-4H-triazino-(2,1-a)-isochinolon-4-on-hydrochlorid-monohydrat.

8. 9,10-Dimethoxy-2-morpholino-6,7-dihydro-4H-triazino-(2,1-a)-isochinolin-4-on-hydrochlorid.

9. 9,10-Dimethoxy-2-mesitylimino-4-(4-methylanilino)-6,7-dihydro-2H-triazino-(2,1-a)-isochinolin-hydrojodid.

10. 9,10-Dimethoxy-2-mesitylimino-4-amino-6,7-dihydro-2H-triazino-(2,1-a)-isochinolin-hydrojodid-monohydrat.

11. 9,10-Dimethoxy-2-mesitylimino-4-(2,4-dimethylanilino)-6,7-dihydro-2H-triazino-(2,1-a)-isochinolin-hydrojodid-monohydrat.

12. Verfahren zur Herstellung von Verbindungen der Formel I

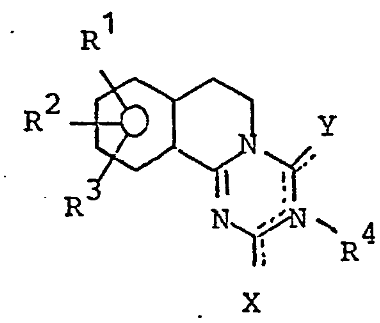

(I)

worin $R^1$, $R^2$ und $R^3$ gleich oder verschieden sein können und Wasserstoff, niederes Alkoxy, Acyloxy oder Halogen sein können, wobei zwei benachbarte Reste $R^1$, $R^2$ oder $R^3$ zusammen eine Alkylendioxygruppe, vorzugsweise eine Methylendioxy- oder Ethylendioxygruppe bedeuten können; $R^4$ ein Elektronenpaar, Wasserstoff oder niederes Alkyl bedeutet;

X Halogen, ein Sauerstoff- oder Schwefelatom, eine Iminogruppe, die mit nieder-Alkyl oder Aryl substituiert sein

kann, Alkylthio, Alkoxy oder $NR^5R^6$, worin $R^5$ und $R^6$ jeweils Wasserstoff, niederes Alkoxy, Amino, niederes Alkylamino, niederes Dialkylamino, Acylamino, niederes Alkoxycarbonylamino, einen stickstoffhaltigen heterocyclischen Rest, niederes Alkyl, $C_3$-$C_6$-Cycloalkyl, niederes Dialkylaminoalkyl, Aralkyl oder heterocyclisch substituiertes niederes Alkyl oder Aryl sein können oder worin $R^5$ und $R^6$ zusammen mit dem Stickstoffatom, an das sie gebunden sind, einen gegebenenfalls substituierten Stickstoffheterocyclus, der ein weiteres Stickstoff- oder Sauerstoffatom enthalten kann, sein können, bedeutet;
Y ein Sauerstoff- oder Schwefelatom, niederes Alkylthio oder $NR^7R^8$, worin $R^7$ und $R^8$ die für $R^5$ und $R^6$ angegebenen Bedeutungen haben, bedeutet,
dadurch gekennzeichnet, daß man eine Verbindung der Formel II

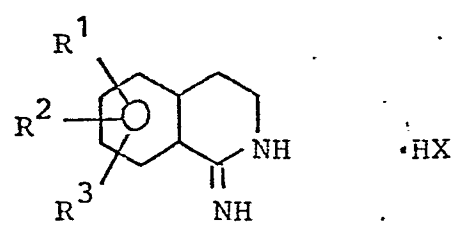

(II)

worin $R^1$, $R^2$ und $R^3$ die oben genannte Bedeutung besitzen und X ein Halogenatom bedeutet, mit einer Verbindung der Formel III

$$O=C=N-\overset{O}{\overset{\|}{C}}-R^6 \qquad \text{(III)}$$

worin $R^6$ eine abspaltbare Schutzgruppe bedeutet, umsetzt zu einer Verbindung der Formel Ia, worin $R^4$ ein Wasserstoffatom und X und Y jeweils ein Sauerstoffatom bedeuten, und gegebenenfalls eine Verbindung der Formel Ia umsetzt mit einem Halogenierungsmittel und anschließend mit einer Verbindung der Formel IV

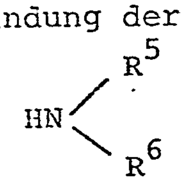

(IV)

worin $R^5$ und $R^6$ die zur Formel I angegebene Bedeutung haben, zu einer Verbindung der Formel Ib, worin $R^4$ ein Elektronenpaar, X die Gruppe $NR^5R^6$ und Y ein Sauerstoffatom bedeutet,
und gegebenenfalls eine Verbindung der Formel Ib mit einem Sulfid umsetzt zu einer Verbindung der Formel Ic, worin $R^4$ ein Elektronenpaar, X die Gruppe $NR^5R^6$ und Y ein Schwefelatom darstellt,
und gegebenenfalls eine Verbindung der Formel Ic, worin $R^6$ Wasserstoff bedeutet, mit einem Alkylhalogenid umsetzt zu einer Verbindung der Formel Id, worin $R^4$ ein Elektronenpaar, X den Rest $=NR^5$ und Y eine Thioalkylgruppe darstellt,
und gegebenenfalls eine Verbindung der Formel Id mit einer Verbindung der Formel V

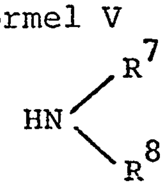

(V)

umsetzt zu einer Verbindung der Formel Ie, worin $R^4$ ein Elektronenpaar, X den Rest $=NR^5$ und Y die Gruppe 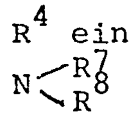 bedeutet.

13. Arzneimittel, gekennzeichnet durch einen Gehalt an einer Verbindung der Formel I in Anspruch 1.

14. Eine Verbindung der Formel I zur Verwendung als Arzneimittel.

0069953

Europäisches Patentamt

# EUROPÄISCHER RECHERCHENBERICHT

Nummer der Anmeldung

EP 82105965.6

| EINSCHLÄGIGE DOKUMENTE | | | KLASSIFIKATION DER ANMELDUNG (Int Cl 3) |
|---|---|---|---|
| Kategorie | Kennzeichnung des Dokuments mit Angabe, soweit erforderlich, der maßgeblichen Teile | betrifft Anspruch | |
| A | EP - A2-0 010 759 (HOECHST)<br>* Zusammenfassung; Patentanspruch 6 *<br>-- | 1,12, 14 | C 07 D 471/04<br>C 07 D 491/147<br>A 61 K 31/53//<br>(C 07 D 471/04<br>C 07 D 221/00<br>C 07 D 251/00) |
| A | DE - A1 - 2 801 289 (HOECHST)<br>* Patentansprüche 1,12 *<br>-- | 1,13, 14 | |
| A | DE - A - 1 922 837 (AGRIPAT)<br>* Seite 1 *<br>---- | 1 | |
| | | | RECHERCHIERTE SACHGEBIETE (Int Cl 3)<br>C 07 D 471/00<br>C 07 D 491/00 |
| | | | KATEGORIE DER GENANNTEN DOKUMENTE<br>X: von besonderer Bedeutung allein betrachtet<br>Y: von besonderer Bedeutung in Verbindung mit einer anderen Veröffentlichung derselben Kategorie<br>A: technologischer Hintergrund<br>O: nichtschriftliche Offenbarung<br>P: Zwischenliteratur<br>T: der Erfindung zugrunde liegende Theorien oder Grundsätze<br>E: älteres Patentdokument, das jedoch erst am oder nach dem Anmeldedatum veröffentlicht worden ist<br>D: in der Anmeldung angeführtes Dokument<br>L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| VIENNA | 16-10-1982 | ONDER |